# EUROPÄISCHE PATENTSCHRIFT

(11) **EP 2 094 641 B1**
(45) Veröffentlichungstag und Bekanntmachung des Hinweises auf die Patenterteilung: **18.06.2014**
(21) Anmeldenummer: 07846876.6
(22) Anmeldetag: 29.11.2007
(51) Int. Cl.: C07C 69/96, A61K 8/37

(54) **DIALKYLCARBONATE VON VERZWEIGTEN ALKOHOLEN UND IHRE VERWENDUNG**
DIALKYL CARBONATES OF BRANCHED ALCOHOLS AND THEIR USE
DIALKYLCARBONATES D'ALCOOLS RAMIFIÉS ET LEUR UTILISATION

(30) Priorität: 08.12.2006 EP 06025406
(43) Veröffentlichungstag der Anmeldung: 02.09.2009
(73) Patentinhaber: Cognis IP Management GmbH, 40589 Düsseldorf (DE)
(72) Erfinder: ANSMANN, Achim, 40699 Erkrath (DE); BOUTTY, Bernd, 40667 Meerbusch (DE); DIERKER, Markus, 40593 Düsseldorf (DE); BRÜNING, Stefan, Dr., 40593 Düsseldorf (DE); KAWA, Rolf, 40789 Monheim (DE)
(86) Internationale Anmeldenummer: PCT/EP2007/010351
(87) Internationale Veröffentlichungsnummer: WO 2008/067946

(56) Entgegenhaltungen:
- EP-A- 0 283 946
- EP-A1- 1 398 366
- EP-A2- 1 083 247
- WO-A-92/10462
- WO-A-97/47583
- WO-A-2005/074864
- DE-A1- 4 125 765
- US-A1- 2005 281 770
- KLEE, S. K. ET AL: "Triggered release by skin pH - novel encapsulation technology for the delivery of personal care actives" SOFW JOURNAL , 132(5), 2, 4-6, 8 CODEN: SOFJEE; ISSN: 0942-7694, Mai 2006 (2006-05), XP002433456

## Beschreibung

### Gebiet der Erfindung

Die Erfindung betrifft Dialkylcarbonate von verzweigten Alkoholen und ihre Verwendung in kosmetischen und/oder pharmazeutischen Zubereitungen.

### Stand der Technik

Im Bereich kosmetischer Zubereitungen für die Haut- und Haarpflege werden vom Verbraucher eine Vielzahl von Anforderungen gestellt: Abgesehen von den reinigenden und pflegenden Effekten, die den Anwendungszweck bestimmen, wird Wert auf so unterschiedliche Parameter wie höchstmögliche dermatologische Verträglichkeit, gute rückfettende Eigenschaften, elegantes Erscheinungsbild, optimaler sensorischer Eindruck und Lagerstabilität gelegt.

Zubereitungen, die zur Reinigung und Pflege der menschlichen Haut und der Haare eingesetzt werden, enthalten in der Regel neben einer Reihe von oberflächenaktiven Substanzen, vor allem Ölkärper und Wasser. Als Ölkörper/Emollients werden beispielsweise Kohlenwasserstoffe, Esteröle sowie pflanzliche und tierische Öle/Fette/Wachse eingesetzt. Um die hohen Anforderungen des Marktes bezüglich sensorischer Eigenschaften und optimaler dermatologischer Verträglichkeit zu erfüllen, werden kontinuierlich neue Ölkörper und Emulgator-Gemische entwickelt und getestet.

Dialkylcarbonate von linearen Alkoholen und ihre kosmetische Verwendung sind aus dem Stand der Technik bekannt, so beschreibt WO 97/47282 die kosmetische Verwendung von Octyl-methyl-carbonat, EP 1 510 199 die kosmetische Verwendung von Distearylcarbonat. Di-n-octylcarbonat ist als kosmetischer Rohstoff unter dem Handelsnamen Cetiol® CC (Cognis Deutschland GmbH & Co. KG) erhältlich.

Ähnliche Carbonate mit einem guten Spreitvermögen werden in speziellen kosmetischen Zubereitungen eingesetzt (Lee, S.K. et al. "Triggered release by skin ph-novel encapsulation technology for the delivery of personal care actives" SÖFW Journal, 132 (5), S. 2, 4-6, 8; Mai 2006).

In der WO 2005/074864 werden Dialkyl(en)carbonate, insbesondere Dihexyl, Dioctyl-, di-(2-ethylhexyl) und Dioleylcarbonate offenbart, die in Zusammensetzungen verwendet werden, um deren sensorische Eigenschaften zu verbessern und insbesondere ein nicht-fettendes Hautgefühl herbeizuführen. Kosmetische Zusammensetzungen mit Diethylhexylcarbonat werden in der Internationalen Anmeldung WO 97/47583 dargestellt.

Die Herstellung von Dialkylcarbonaten ist in WO 97/47583 beschrieben. Die nach dem dort beschriebenen Verfahren erhältlichen symmetrischen Dialkylcarbonate enthalten als Nebenprodukte z.B. 2-Ethyl-1-hexyl-methylcarbonat oder 2-Butyl-1-Octyl-methylcarbonat. Eine kosmetische Verwendung dieser Verbindungen wird nicht erwähnt. EP 1 083 247 beschreibt verschiedene kurzkettige verzweigte Dialkylcarbonate und ihre Eignung zum Waschen von Metalloberflächen.

Profil verfügen und in eine Vielzahl kosmetischer Formulierungen einarbeitbar sind, beispielsweise als Ölkörper/Emollient oder als Konsistenzgeber. Die Rohstoffe sollten sowohl in W/O als auch in O/W Formulierungen einarbeitbar sein und insbesondere mit kristallinen UV-Filtern, Pigmenten, Antiperspirantlen Salzen sowie Silikonen kompatibel sein. Von besonderem Interesse war es Rohstoffe bereit zu stellen, welche ein gutes oder verbessertes Lösevermögen für Pigmente oder anorganische UV-Filter aufweisen.

# das Dialkylcarbonat der Formel (I) R¹O-CO-OR² (I), wobei R¹ und R² ein 2- Propyl-1-heptyl-Rest ist Überraschenderweise wurde gefunden, dass # diese Aufgabe erfüllt und insbesondere zu sensorisch leichten Produkten führt, Überraschenderweise eignen sich die Dialkylcarbonate der vorliegenden Erfindung insbesondere um polare Verbindungen, insbesondere Pigmente oder anorganische UV-Filter zu lösen. Somit wird eine leichtere Formulierbarkeit dieser Wirkstoffe in kosmetischen und/oder pharmazeutischen Zubereitungen erreicht.

### Beschreibung der Erfindung

Dialkylcarbonate sind Ester der Kohlensäure der allgemeinen Formel O=C(OR)₂, wobei R einen Alkyl-Rest bedeutet. Die Bezeichnung der Dialkylcarbonate erfolgt entweder, indem die Alkylsubstituenten dem Begriff "Carbonat" voran stellt oder als Kohlensäure"alkyl"ester. So z.B. O=C(OC₈H₁₇)₂ als Dioctylcarbonat oder Kohlensäuredioctylester oder z.B. O=C(OC₂H₆)(OC₈H₁₇) als Ethyl-octyl-carbonate oder als Kohlensäure-Ethyl-Octylester. Im Folgenden wird für die Dialkylcarbonate die Formel R-O-CO-O-R verwendet.

Gegenstand der Erfindung rind Dialkylcarbonate der Formel (I) R¹O-CO-OR² (I), wobei R¹ und R² ein 2- Propyl-1-heptyl-Rest ist.

Ein weiterer Gegenstand der Erfindung betrifft die Verwendung aller vorgenannten Dialkylcarbonate in kosmetischen Zubereitungen. Überraschenderweise wurde festgestellt, dass sich die erfindungsgemäßen Dialkylcarbonate besonders eignen zur Herstellung von kosmetischen Zubereitungen, sie eignen sich insbesondere als Ölkörper/Emollients und/oder Konsistenzgeber in kosmetischen Zubereitungen. Die erfindungsgemäßen Dialkylcarbonate eignen sich weiterhin zur Herstellung von pharmazeutischen Zubereitungen, wobei die Dialkylcarbonate als technische Hilfsstoffe, wie z.B. Ölkörper eingesetzt werden. Die erfindungsgemäßen Dialkylcarbonate können zur Herstellung von kosmetischen Zubereitungen, wie beispielsweise Haarshampoos, Haarlotionen, Schaumbäder, Duschbäder, Cremes, Gele, Lotionen, alkoholische und wässrig/alkoholische Lösungen, Emulsionen, Wachs/ Fett-Massen, Stiftpräparaten, Pudern oder Salben dienen. Diese Mittel können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten. Bevorzugt ist die Verwendung der Dialkylcarbonate als Ölkörper.

Die erfindungsgemäßen Dialkylcarbonate können in kosmetischen Formulierungen als sog. light emollients' verwenden werden, um spezielle Eigenschaften, wie z.B. Spreitverhalten oder Flüchtigkeit einzustellen. Die erfindungsgemäßen Dialkylcarbonate ermöglichen weiterhin viskositätsstabile kosmetische Formulierungen herzustellen.

### Herstellung

Die Herstellung der Dialkylcarbonate erfolgt nach dem Fachmann bekannten Verfahren beispielsweise durch Umesterung niederer Dialkylcarbonate, wie beispielsweise Dimethylcarbonat, Diethylcarbonat, Dipropylcarbonat oder Dibutylcarbonat mit den entsprechenden Alkoholen oder Alkoholgemischen in Gegenwart eines Katalysators, wie beispielsweise in WO 97/147583 beschrieben.

Andere Herstellverfahren sind beschrieben in M. Dierker: Lipid Technology, 2004,16(6), S.130-134.

### Vergleichsbeispiele

### Beispiel 1: Herstellung von Hexyldecyl-methylcarbonat

1350g (15 mol) Dimethylcarbonat wurden mit 14g Natriummethylat (30%ig in Methanol) in einer 4 Rührapparatur mit Tropftrichter vorgelegt und auf 80°C aufgeheizt. Dann wurde 699g (1,5 mol) Hexyldecanol über einen Zeitraum von 2 Stunden zugetropft und weitere 9 Stunden 80°C nachgerührt. Entstehendes Methanol wurde währenddessen über einen Destillationsaufsatz aus der Reaktionsmischung abdestilliert. Die GC Auswertung des Rohproduktes ergab ein Zusammensetzung von: 4% Hexyldecanol, 79% asymmetrisches Hexyldecyl-methyl-carbonat und 13% symmetrisches DiHexyldecylcarbonat.
Der pH-Wert wurde mit 14g H₃PO₄ (85%ig) auf pH 3-4 eingestellt und überschüssiges Dimethylcarbonat abdestilliert. Das ausgefallene Natriumphosphat wurde abgesaugt und das Produkt fraktioniert. Das Reaktionsprodukt Hexyldecyl-methyl-carbonat fällt als farbloses Öl mit einem Siedepunkt von 125-125°C bei 0,4 mbar an.

### Beispiel 2: Formulierungen mit Hexyldecyl-methylcarbonat

Die nachfolgenden kosmetischen Rezepturen wurden mit dem nach Beispiel 1 hergestellten Hexyldecylmethylcarbonat hergestellt. Alle Angaben sind in Gew.-%

| Rezeptur Nr. | 1 | 2 |
|---|---|---|
| Handelsname (INCI Bezeichnung) | | |
| Dehymuls®LE (PEG-30-Dipolyhydroxystearate) | 5,00 | 4,00 |
| Lameform®TGI (Polglyceryl-3-Diisostearate) | 0,00 | 2,00 |
| Hexyldecyl-methylcarbonat | 20,00 | 20,00 |
| MgSO4*7H2O | 1,00 | 1,00 |
| Glycerin 99,5%ig | 5,00 | 5,00 |
| Formalinlsg. 37%ig | 0,15 | 0,15 |
| Wasser dest. | ad 100 | ad 100 |

| Rezeptur Nr.: | 3 | 4 | 5 |
|---|---|---|---|
| Handelsname (INCI Bezeichnung) | | | |
| Emulgade® PL 68/50 (Cetearyl glucoside (and) Cetearylalcohol) | 4,50 | 0,00 | 0,00 |
| Eumulgin® VL75 (Lauryl glucoside (and) Polyglyceryl-2-Dipolyhydroxystearate (and) Glycerin | 0,00 | 4,50 | 0,00 |
| Eumulgin® B2 (Ceteareth-20) | 0,00 | 0,00 | 2,00 |
| Hexyldecyl-methylcarbonat | 16,00 | 16,00 | 16,00 |
| Carbopol® 980 (Carbomer) | 0,00 | 0,30 | 0,00 |
| Lanette® O (Cetearylalcohol) | 0,00 | 0,00 | 5,00 |
| KOH (20%ig) | 0,00 | 0,60 | 0,00 |
| Glycerin 99,5%ig | 3,00 | 3,00 | 3,00 |
| Formalinlsg. 37%ig | 0,15 | 0,15 | 0,15 |
| Wasser dest. | ad 100 | ad 100 | ad 100 |

## Patentansprüche

1. Dialkylcarbonat der Formel (I) R1O-CO-OR2 (I), wobei R1 und R2 ein 2- Propyl-1-heptyl-Rest ist.

2. Verwendung des Dialkylcarbonats nach Anspruch 1 in kosmetischen und/oder pharmazeutischen Zubereitungen.

3. Verwendung nach Anspruch 2 als Ölkörper oder als Konsistenzgeber.

## Claims

1. Dialkyl carbonate of the formula (I) **R1O-CO-OR2 (I)**, wherein R1 and R2 are a 2-propyl-1-heptyl radical.

2. Use of the dialkyl carbonate according to Claim 1 in cosmetic and/or pharmaceutical preparations.

3. Use according to Claim 2 as oil substances or as agents which impart consistency.

## Revendications

1. Dialkylcarbonate de formule (I) R1O-CO-OR2 (I), dans laquelle R1 et R2 sont un radical 2-propyl-1-heptyle.

2. Utilisation du dialkylcarbonate selon la revendication 1 dans des préparations cosmétiques et/ou pharmaceutiques.

3. Utilisation selon la revendication 2 en tant que corps huileux ou en tant que donneur de consistance.
